# EUROPEAN PATENT APPLICATION

(11) **EP 2 982 336 A1**
(43) Date of publication of application: **10.02.2016**
(21) Application number: 14179688.8
(22) Date of filing: 04.08.2014
(51) Int. Cl.: A61F 2/24

(54) **Mitral valve prosthesis, particularly suitable for transcatheter implantation**

(71) Applicant: Alvimedica Tibb Ürünler San. Ve Dis Tic. A.S., 34540 Itanbul (TR)
(72) Inventor: Minoletti, Francesco, 10149 Torino (IT); Antoniotti, Matteo, 10036 Settimo Milanese (Torino) (IT); Vallana, Franco, 10123 Torino (IT); Curcio, Maria, 13040 Saluggia (Vercelli) (IT)
(74) Representative: Gerbino, Angelo

(57) **Abstract**

A radially expandable and collapsible armature (9) for a prosthetic mitral valve comprises a composite annular element (14), including a first support ring (14a) and a self-securing ring (14b), which is radially expandable when subjected to an axial strength, and an anchoring cup-shaped frame (10), comprising a plurality of expandable meridian elements (10b) connected by a lower expandable arched elements (10c), in such a way that the latters have a radius (r) smaller than the radius (R) of the formers.

## Description

### Technical field

The present invention relates, in general, to heart valve prostheses; in particular, the invention relates to a mitral valve prosthesis.

### Background of the invention

The need to provide prosthetic mitral valves, which can effectively replace the native valves, when there are problems in the efficiency of the same, is increasingly perceived.

In particular, the mitral valves prosthesis are oriented, although not exclusively, to elderly patients more prone to degenerative syndromes which involve difficulties in the dynamics of closing of the valve, with impairment of the physiological hemodynamic and consequent heart failure, reflux, prolapse and phenomena of heart enlargement, due to the reduced efficiency in maintaining a sufficient blood flow.

In this sense, the attention in the realization of a mitral valve prosthesis focuses in the search for solutions designed to ensure an optimal closing dynamics of the valve, usually affected by prolapse phenomena impairing the correct closing of the leaflets.

The prosthesis is therefore called to be firmly and effectively anchored to the walls of the left atrium and the left ventricle, providing a sufficiently stable anchoring force to counteract the pressure exerted by the ventricle contraction, the mitral valve having to prevent the backflow of blood to the atrium and the pulmonary veins, so as to allow the inflow into the aorta.

However, the solutions known up to date do not allow to efficiently contrast such a pressure, which could force the mitral valve to displace with consequent leakage, this is because the existing prosthesis do not have anchoring structures morphologically suitable to ensure the positional stability of the valve *in situ.*

In particular, the foregoing applies to the valve prosthesis for percutaneous implantation, where no suture can be applied and in which the need to have a substantially collapsible and elastic structure contrasts with the need to ensure an adequately stable anchoring to the walls of the valve site.

Examples of a cardiac prosthesis, more or less suitable for the implantation as a mitral valve, are known for example from the document US 2011/022478 (which discloses a device to be coupled to a native atrioventricular valve, including a support frame, shaped so as to define a downstream skirt, and an elongated anchoring member, positioned around the downstream skirt in a subvalvular space, such that the anchoring member presses the leaflets of the native valve against the downstream skirt, thereby anchoring the prosthetic valve to the native valve), from the document EP 1 690 515 (which discloses a valve for percutaneous implantation, comprising an armature for anchoring the valve prosthesis in the implantation site and for supporting a set of leaflets, said armature comprising two annular parts, with the capacity of projecting radially so as to engage anatomical formations and secure the prosthesis within the valve site), from the document WO 2008/091515 (which describes a compressible and expandable stent assembly for implantation in a body lumen such as a mitral valve, including stent barrels that are shaped and sized so that they allow for normal operation of adjacent heart structures, where the stent barrels can include a cylinder with a tapered edge), from the document US 2011/137397 (wherein the prosthetic mitral valve includes a radially compressible main body, a one-way valve portion, and at least one ventricular anchor coupled to the main body, in such a way that a space is provided between the outer surface of the main body and the ventricular anchor for receiving a native mitral valve leaflet, the prosthetic valve further including an atrial sealing member adapted for placement above the annulus of the mitral valve), from the document US 2009/125098 (which discloses an implantable endoluminal prosthesis for replacing a damaged aortic valve, including a balloon-expandable stent, a tubular conduit that extends into the ascending aorta, and a self-expanding stent, coupled to provide unidirectional flow of fluid into the aorta and further into the coronary arteries), from the document US 2010/312333 (which discloses an apparatus comprising a barbell-shaped, expandable anchoring member including first, second, and main body portions extending between the end portions, in such a way that the main body portion includes a channel the first and second end portions are sized to respectively contact the superior and inferior aspects of the native cardiac valve annulus, when the expandable anchoring member is in an expanded configuration, the apparatus also including an expandable support member operably disposed within the main body portion of the expandable anchoring member, and a prosthetic cardiac valve secured within the expandable support member), from the document WO 2004/016200 (which discloses a valve prosthesis, such as an artificial venous valve, having a support frame and leaf structure comprising one or more leaflets in which the outer edge of each leaflet engages the inner circumference of the bodily passageway along a serpentine path urged against the passageway by an expandable frame, while the inner edges move in response to fluid to restrict retrograde flow; optionally, one or more elements can extend from the support frame/leaf structure to provide centering support and/or protection from the leaflet adhering to the vessel wall, the centering support structure comprising a second or third expandable frames attached to and extending from the proximal and/or distal ends of main valve structure and support frame), and from the document US 2005/182486 (which discloses an apparatus including a base portion, operative to maintain a circumferential dimension thereof, and a plurality of fingers extending axially and radially outwardly from the first end of the support element, so that a heart valve can be located within the apparatus to provide a prosthesis that can be suturelessly implanted at an annulus of a patient).

It is to be understood that the reference to any one of the preceding prior art documents must not be interpreted as limiting the scope of the present invention.

Although the independence achieved between the support structures of the biological valve, and the anchoring structures, decreases the distortion of the biological valve, which principle is valid for the valve prosthesis according to the present invention, the structures contemplated in the prior art do not enable an effective positioning of the mitral valve prosthesis as replacement of the defective native valve.

### Summary of the invention

One purpose of the present invention is to provide a mitral valve prosthesis suitable to effectively anchoring in the same position of the defective native valve, contrasting the pressure exerted by the contraction of the ventricle on the closed valve, and allowing the blood to flow from the atrium to the ventricle when the valve is open.

For achieving this result, a mitral valve prosthesis according to the present invention includes an armature adapted to support and contain the prosthetic valve, allowing in a particularly effective way, although not exclusively, the implantation via percutaneous approach (via transcatheter) or less invasive surgical procedures (via transapical).

The armature, as will be better appreciated in the description as follows, includes an expanded cup-shaped section, which abuts against the walls of the atrium, and helps to keep the prosthesis in place when it is subject to forces due to blood flow that opens it and passes through; furthermore, in order to better ensure the anchorage, a composite ring engages the *anulus* and partially the atrium, expanding radially when the contraction of the ventricle generates a closing pressure on the valve, so as to strengthen the anchoring action of the armature 9 in the valve site.

Furthermore, an expandable arched element holds in a dilated configuration the expanded section, holding it in abutment against the walls of the atrium which, being constituted by tissue with poor muscle fibers, provides a relatively stable hooking surface.

A lower ring may provide an attachment basis for support pillars of the valve leaflets.

In this way, the prosthetic valve is steadily retained in place thanks to the combination of the anchoring actions exerted by the various sections of the support armature.

The above and other objects and advantages are achieved, according to one aspect of the invention, by an armature for a mitral valve prosthesis having the features defined in claim 1. Preferred embodiments of the invention are defined in the dependent claims.

### Brief description of the drawings

The structural and functional features of some preferred embodiments of an armature for a mitral valve prosthesis according to the invention are to be described in the following. Reference is made to the attached drawings in which:
- Figure 1 shows a front view of an armature for a mitral valve prosthesis, according to one embodiment of the present invention;
- Figures 2A and 2B respectively show a part of the armature of Figure 1 and the corresponding cross-sectional view;
- Figure 3 shows an armature according to an alternative embodiment of the present invention;
- Figure 4 shows a plane representation of the armature of Figure 1;
- Figure 5 shows an armature according to an alternative embodiment of the present invention;
- Figure 6 shows a plane representation of the armature of Figure 5; and
- Figures 7A-7E show schematic views of a grid element composing a self-securing ring, according to an alternative embodiment of the present invention.

### Detailed description of the invention

Before explaining in detail a plurality of embodiments of the present invention, it should be clear that the invention is not limited in its application to the details of construction and to the configuration of the components described in the following or illustrated in the drawings. The invention is able to assume other embodiments and of being implemented or realized practically in different ways.

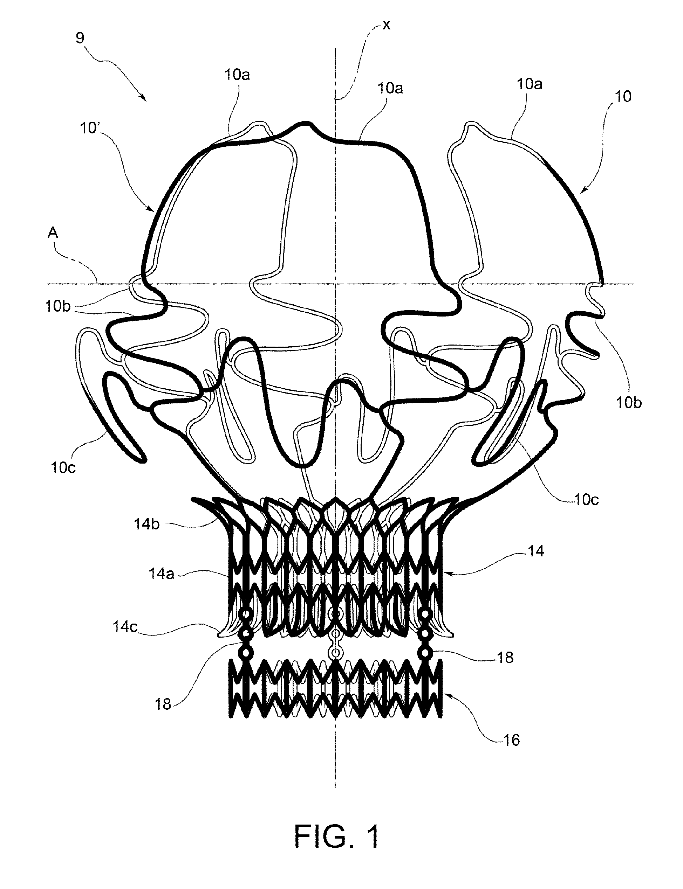

Referring initially to Figure 1, an armature 9 for a heart valve prosthesis, for the functional replacement of a diseased mitral valve, has a shape substantially comparable to a solid of revolution with respect to a longitudinal axis x.

Said armature 9 includes an expanded section or cup-shaped anchoring frame 10, able to engage the walls of the left atrium of the heart. Such cup-shaped frame 10 may be considered substantially as a part of a sphere or a globe.

The cup-shaped anchoring frame 10 is mechanically connected to a composite annular element 14, which simultaneously provides a support for a prosthetic valve (not shown in the drawings) and helps to strengthen the action of anchorage on the walls of the heart.

Conveniently, a lower annular support element 16 provides a second support section for support pillars 18, which extend in the axial direction with respect to the armature 9, support pillars 18 on which the commissures of the biological valve are sewn.

The armature 9 is globally collapsible, i.e. any part thereof is able to expand from a first contracted position to a second expanded position, when placed in situ.

In particular, the cup-shaped anchoring frame 10 includes a plurality of arched structures or expandable meridian elements 10b, which in the expanded configuration follow the inner surface of the atrium, helping to strengthen the anchoring action of the prosthesis to the atrium. Therefore, unlike the annular composite element 14 and the lower annular support element 16, whose radial expansion is substantially cylindrical, the expandable meridian elements 10b mutually diverge along the outer surface of a dilating globe.

The expandable meridian elements 10b may have free upper ends, or be connected in pairs by an upper crown 10', formed by at least one arched abutment element 10a (in the embodiment illustrated in Figure 1, having the shape of a petal or a cusp).

Figure 1 also illustrates an embodiment according to which there are three pairs of expandable meridian elements 10b, each pair being connected by a respective arched abutment element 10a.

The cusps 10a of the upper crown 10' may extend along a substantially axial direction, or have a curvature towards the inside of the expanded section 10, to help the positioning and adaptation of the armature 9 to the wall of the atrium.

In Figure 1, an expandable arched element 10c can be noted, which circumferentially connects at least a pair of meridian elements 10b. This expandable arched element 10c supports the expansion of the cup-shaped anchoring frame 10, ensuring the proper expansion thereof when the armature 9 is placed *in situ.*

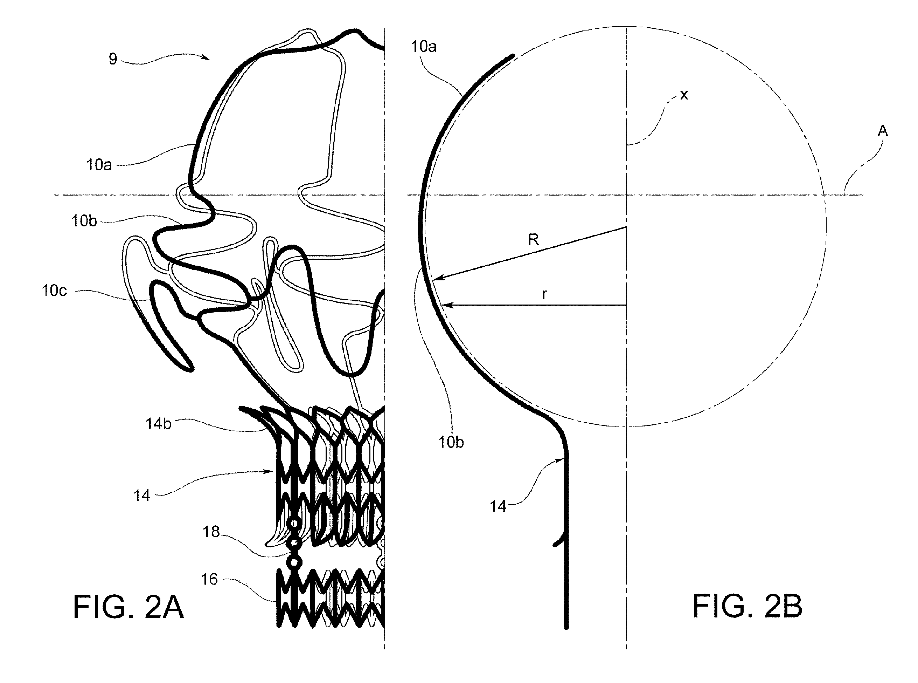

The expandable arched element 10c is placed substantially in proximity of the composite annular element 14, and below the most radially expanded section of the globe or virtual sphere of envelop of the cup-shaped anchoring frame 10; this, because such a position consolidates and reinforces the anchoring action exerted by the arched structures or meridian elements 10b, without interfering with the possibility of the crown 10' to adapt to the walls of the atrium. More precisely, the expandable arched element 10c shall have a radius r (shown in Figure 2B) smaller than a radius R of a virtual circle encompassing the upper parts of the meridian elements 10b along a plane transverse with respect to the longitudinal axis x of the armature 9. The radius r of the expandable arched element 10c is definable as the radius of curvature thereof.

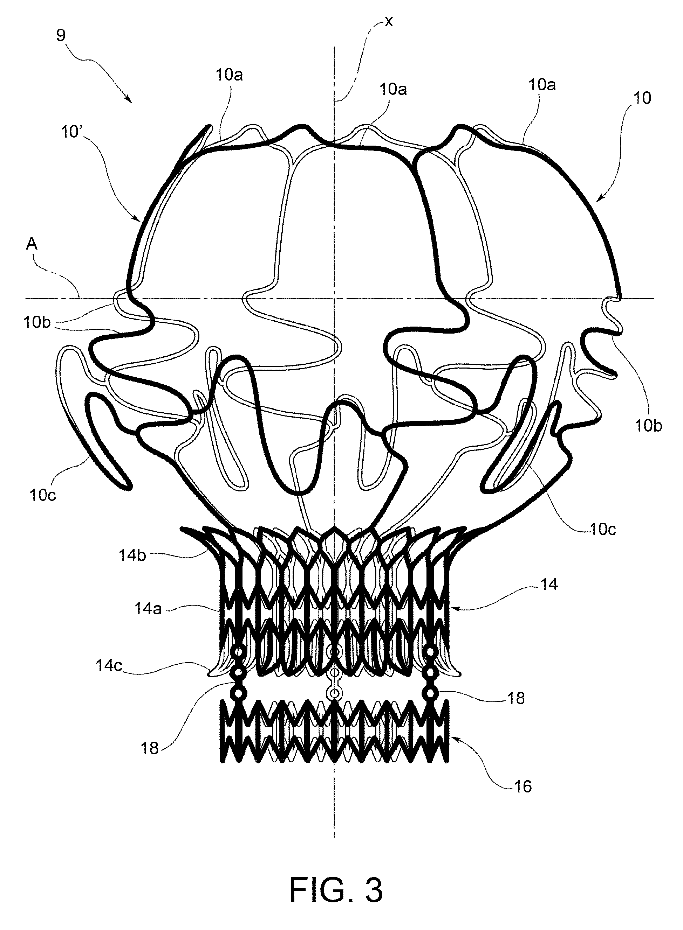

If the cup-shaped anchoring frame 10 is an hemisphere or a globe (as shown, for example, in Figures 1 and 3), the radius R of the virtual circle would correspond with a virtual equator A of the globe or hemisphere enveloping said cup-shaped anchoring frame.

The expanded structure 10 is particularly effective where the blood flow is directed from the atrium to the ventricle (i.e., when the flow of blood passes through the valve in the open configuration). However, it further balances the pressure that the contraction of the ventricle exerts on the valve, when the latter is in the closed position.

The annular composite element 14 comprises a plurality of sections 14a, 14b, and is able not only to offer a support to the commissures of the valve leaflets, but also to exert a radial anchoring action on the walls of the valve site.

In particular, a section or first support ring 14a, on which the support pillars 18 anchor themselves, is axially surmounted by a self-securing ring 14b, which has a reticular structure such that, for an axial thrust exerted for example by the first support ring 14a upwardly (that is, when the pressure exerted by the contraction of the ventricle tends to apply a high thrust as the valve is to be closed), said self-securing ring 14b undergoes a radial expansion which further pushes against the valve site and strengthens the ability of the armature 9 to resist stably to axial stresses that would tend to displace from the valve site.

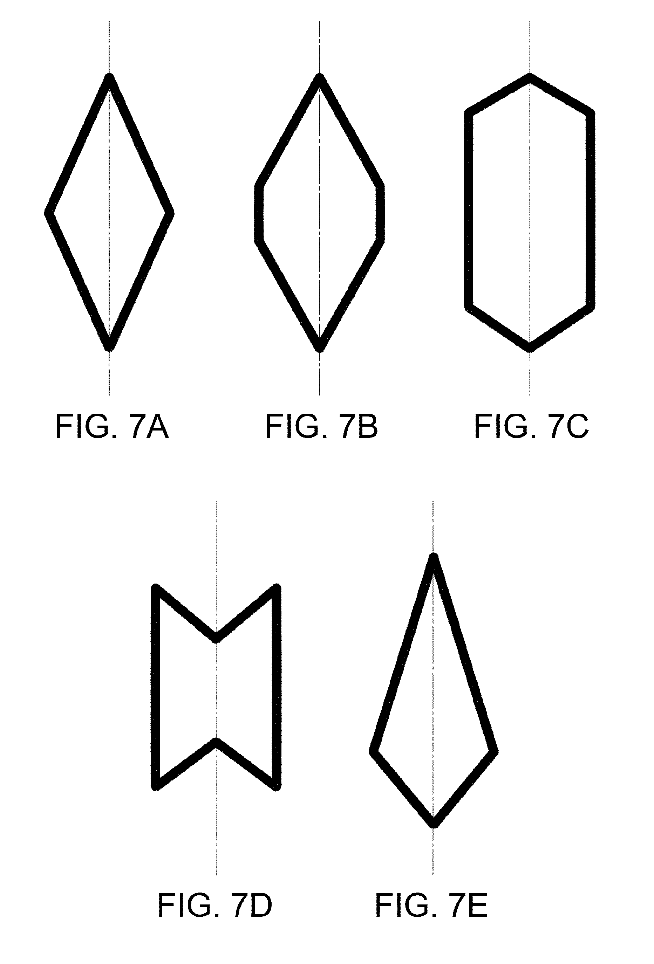

Conveniently, in order to radially expand under a generic strength, the self-securing ring 14b has a reticular structure formed by several stripes of juxtaposed elements, which are geometrically shaped so as to be symmetric with respect to an axis parallel to the longitudinal axis x of the armature 9. For example, such elements may be designed in the shape of circles, ellipsis, or polygons like rhombuses or diamonds (in Figures 7A-7E are shown different possible shapes for the elements composing the reticular structure of the self-securing ring 14b).

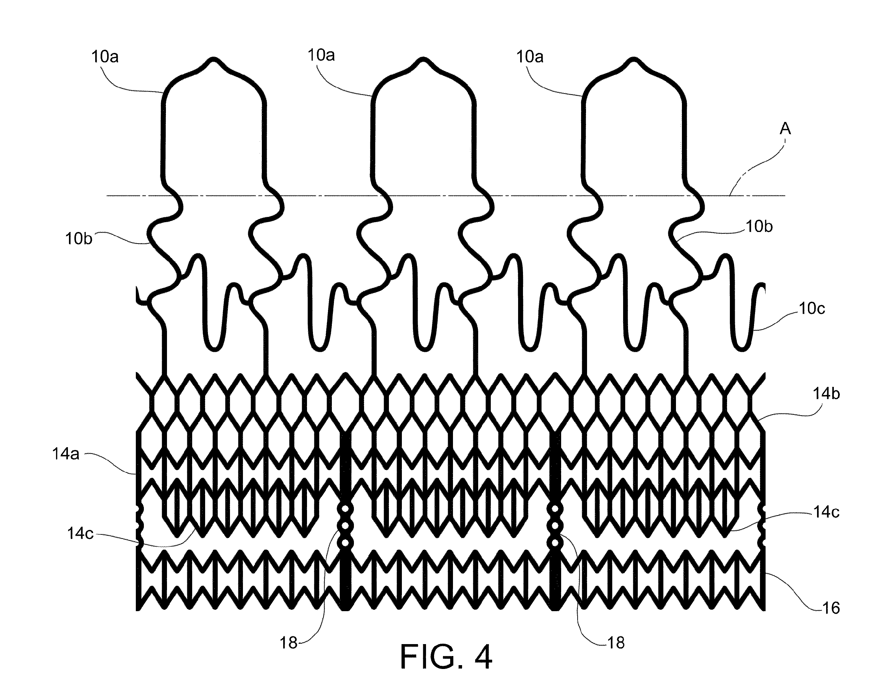

In the embodiment illustrated in the following, the self-securing ring 14b has a grid structure formed by two stripes of alternating hexagons, as illustrated in particular in Figure 4, so that an axial stress generates an expansion of the grid in the radial direction.

According to one embodiment of the invention, the first support ring 14a and the self-securing ring 14b have grid structures having a mesh of different shapes and sizes (as in the example illustrated herein below).

According to an alternative embodiment (not shown), the self-securing ring 14b may lack of an angular sector, in order to better accommodate the armature 9 in the valve site, without interfering with the neighboring aortic valve site.

Conveniently, the composite annular element 14 also includes a flared ring 14c, in a position axially lower than that of the first support ring 14a, to ensure a further abutment against the valve site, and to further balance the axial counterthrusts which would tend to displace the armature from the valve site.

Figures 2A and 2B also show a section of the armature 9, illustrated in Figure 1, with the intent to better show the curvature given to the upper anchoring crown 10', to the meridian elements 10b and to the substantially cylindrical portion of the armature, comprising the composite annular elements 14 and lower support 16. Figure 2B, in particular, shows the longitudinal axis x of the armature 9.

Throughout the present description and claims, the terms and the expressions indicating position and orientation, such as "longitudinal", "axial" or "radial", must be referred to the longitudinal axis x.

Figure 3 shows an alternative embodiment of the armature 9 for a mitral valve prosthesis, according to the present invention. Unlike the embodiment illustrated in Figure 1, the upper crown 10' comprises a succession of bridges or cusps 10a between the meridian elements 10b, such as to provide a continuous abutment structure against the upper walls of the atrium.

As already contemplated in the prior art, the meridian elements 10b are sinusoidal or zigzagging formations, which may allow a more balanced and less sharp abutting against the walls of the atrium.

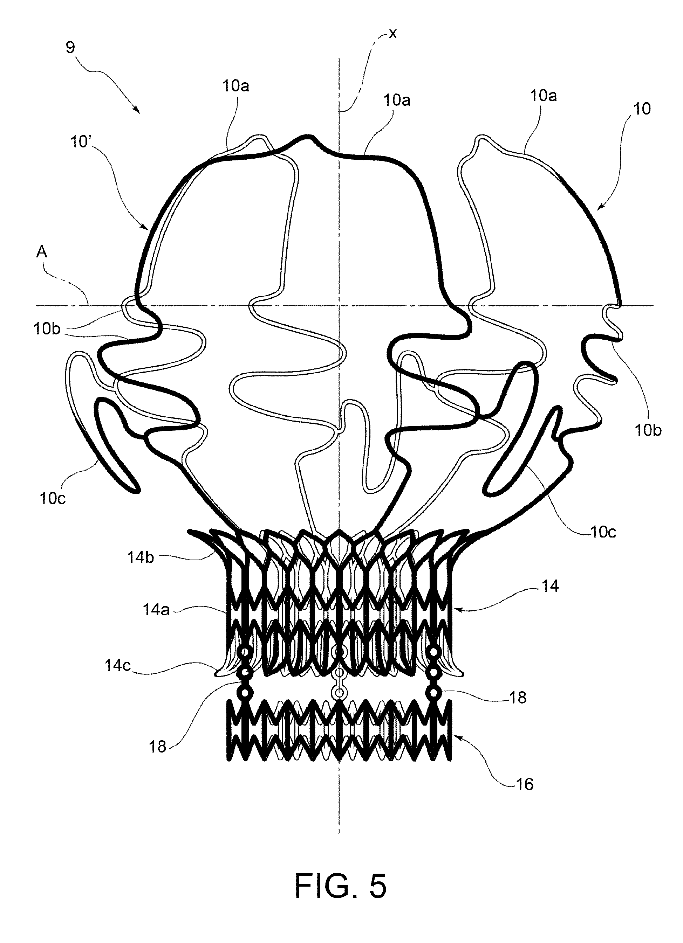

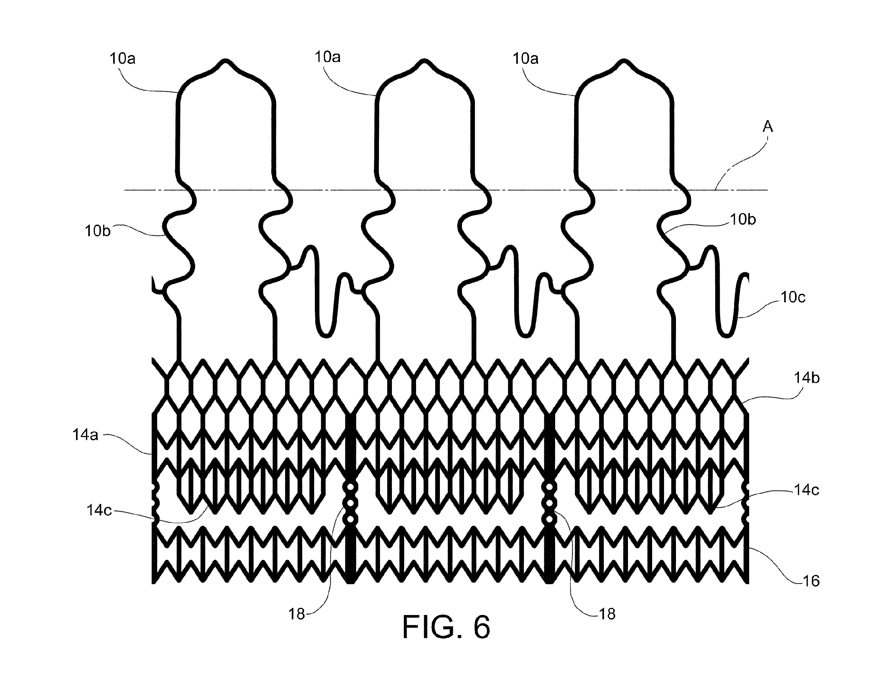

In the example shown here, the expandable arched element 10c connects all the meridian elements 10b, thus realizing a circumferential, substantially seamless, connection. According to an embodiment shown by way of example in Figures 5 and 6, it is possible that the reinforcing ring is actually formed by a plurality of arched sections that connect only some pairs of meridian elements 10b (for example in the number of three, preferably spaced apart by 60°), for example the pairs that in Figure 1 are not mutually connected through the cusps of the crown 10a. The number and the circumferential spacing of the arched sections of the expandable arched element 10c can therefore be determined as preferred.

Finally, Figure 4 shows a plane representation of the armature 9, in which it is possible to distinguish the different design of the meshes forming, for example, the sections of the composite ring 14, as well as a preferred embodiment of the flared ring 14c, which in the example provided herein below consists of three arched sections spaced by 120°. Also in this case, the arrangement and the number of arched sections of the segment 14c are to be considered non-limiting, as the person skilled in the art is able to select a different number and different arrangements of the strokes, also without interruption.

Various aspects and embodiments of the armature for a mitral valve prosthesis according to the present invention have been described. It is understood that each embodiment can be combined with any other embodiment. The invention, moreover, is not limited to the embodiments described, but can be varied within the scope of the invention as defined in the attached claims.

## Claims

1. An armature (9) for a prosthetic mitral valve, having a longitudinal axis (x) and being radially expandable and collapsible, said armature comprising:
- a composite annular element (14), comprising:
- a first support ring (14a); and
- a self-securing ring (14b), axially juxtaposed to said first support ring (14a), and having a radially expandable grid structure suitable to exert an anchoring force in the radial direction when subjected to an axial strength;
- an anchoring cup-shaped frame (10), inferiorly connected to said composite annular element (14), and comprising:
- a plurality of expandable meridian elements (10b), circumferentially spaced, whose upper parts are substantially on a circle having a radius (R), when the armature (9) is in an expanded configuration; and
- an expandable arched element (10c), connecting at least one pair of circumferentially consecutive meridians element (10b), said expandable arched element (10c) having a radius (r) smaller than the radius (R) of said virtual circle;
- a plurality of support pillars (18), axially extended and connected at the top to said first support ring (14a).

2. An armature according to claim 1, wherein at least one arched abutment element (10a) connects superiorly a corresponding pair of meridian elements (10b).

3. An armature according to claim 1 or 2, wherein the support pillars (18) are connected at the bottom to a lower annular support element (16).

4. An armature according to any one of the preceding claims, wherein each pair of circumferentially consecutive meridian elements (10b) is connected superiorly by respective arched abutment elements (10a), forming an upper crown (10').

5. An armature according to any one of the preceding claims, in which each pair of circumferentially consecutive meridian elements (10b) is connected circumferentially through the expandable arched element (10c), which is annular.

6. An armature according to any one of the preceding claims, wherein the grid structure of the self-securing ring (14b) is made by elements which are geometrically shaped so as to be symmetric with respect to a respective axis parallel to the longitudinal axis (x) of the armature (9).

7. An armature according to any one of the preceding claims, wherein the grid structure of the self-securing ring (14b) has a hexagonal mesh pattern.

8. An armature according to any one of the preceding claims, wherein the first support ring (14a) and the self-securing ring (14b) have a different grid structure.

9. An armature according to any one of the preceding claims, wherein the self-securing ring (14b) is at least partially radially outwardly flared.

10. An armature according to any one of the preceding claims, wherein a flared ring (14c) is inferiorly juxtaposed in the axial direction to the first support ring (14a).

11. An armature according to any one of the preceding claims, wherein the self-securing ring 14b is open.

12. A mitral valve prosthesis, comprising an armature according to any one of the preceding claims.
